# EUROPÄISCHE PATENTSCHRIFT

(11) **EP 3 366 253 B1**
(45) Veröffentlichungstag und Bekanntmachung des Hinweises auf die Patenterteilung: **25.12.2024**
(21) Anmeldenummer: 18158021.8
(22) Anmeldetag: 06.09.2012
(51) Int. Cl.: A61B 18/20, A61N 7/00, A61H 23/00

(54) **EINRICHTUNG ZUR BIOLOGISCHEN GEWEBEERNEUERUNG**
DEVICE FOR BIOLOGICAL TISSUE REGENERATION
DISPOSITIF DESTINÉ À LA RÉGÉNÉRATION BIOLOGIQUE DE TISSU

(30) Priorität: 11.05.2012 EA 201200845
(43) Veröffentlichungstag der Anmeldung: 29.08.2018
(62) Teilanmeldung aus: 12876284.6
(73) Patentinhaber: Khomchanka, Uladzimir Valiantinavich, Minsk 220006 (BY); Troshkin, Aleksandr, Bratislava 81109 (SK); Frangulova, Yulia, Bratislava 81109 (SK)
(72) Erfinder: Khomchanka,Uladzimir Valiantinavich, Minsk 220006 (BY); Gorbach, Dmitry Vladislavovich, Minsk 220102 (BY); Sukhadolau, Aliaksandr Valerjavich, Minsk 220117 (BY)
(74) Vertreter: Jeck, Anton

(56) Entgegenhaltungen:
- WO-A2-2012/018391
- US-A- 5 423 803
- US-A- 5 725 482
- US-A- 6 086 580
- US-A1- 2002 058 890
- US-A1- 2002 161 357
- US-A1- 2005 085 748
- US-A1- 2009 069 741
- US-A1- 2010 204 619
- US-B1- 6 595 934

## Beschreibung

Die Erfindung betrifft eir Vorrichtung zur biologischen Gewebeerneuerung nach dem Oberbegriff des Anspruchs 1.

Die Erfindung ist in der Medizin und auch in der Chirurgie, darunter auch in der Schönheitschirurgie, einsetzbar. Somit ist die Erfindung z. B. zur Behandlung von trophischen und lange nicht heilenden Geschwüren, Dekubitalgeschwüren, Brandverletzungen, Narben usw. geeignet. Ein weiterer Anwendungsbereich ist die Verjüngung der biologischen Gewebe, darunter der Haut, mit unterschiedlicher Lokalisation. Das mit der Vorrichtung zur Verfügung gestelte Verfahren beruht auf einer Übertragung einer nichtmechanischen Energie, insbesondere akustischen Wellenenergie, in die biologischen Gewebe des menschlichen Körpers. Die Erfindung betrifft verschiedene Ausgestaltungen einer Einrichtung, die die zur Ausführung des Verfahrens benötigte Wellenenergie generiert.

So ist z. B. ein Hautverjüngungsverfahren mittels Ablation oder Vaporisation der Oberhaut mit Hilfe einer CO2-Kohlensäuregaslaserstrahlung mit 10,6 µm langen Wel-len oder einer Er-Laserstrahlung (Er: YAG) mit 2,94 µm langen Wellen (Palomar 2940 Fractional Laser, Deka SmartXide DOT, Candela C02RE) [1] bekannt. Der Heileffekt beruht in diesem Fall auf einer Verdampfung in der Oberhaut mit geringfügigem Wärmeschaden für die tiefen Hautschichten. Das führt zu keiner Volldestruktion aller Hautschichten und ruft ein neues Zellwachstum hervor. Die Mängel dieses bekannten Verfahrens sind seine hohe Traumatisierung, eine lange Rehabilitationsdauer und ein Vorhandensein einer Wundoberfläche. Dies verursacht ein hohes Ansteckungsrisiko, eine Dolenz sowohl während der Behandlung als auch im Laufe der Regenerationszeit, ein Risiko einer Pigmentierungsänderung und einer Bildung von narbigen Hautveränderungen. Darüber hinaus ist das Verfahren zur Anwendung an beweglichen Körperteilen, wie Hals, Augenlidern usw., nicht geeignet, weil der Heilungsverlauf der Wundoberfläche eine Unbeweglichkeit des Behandlungsfelds voraussetzt.

Aus dem Stand der Technik ist auch ein Verfahren einer nichtinvasiven Fotoverjüngung bekannt. Dabei dringt Strahlung tief in die Haut hinein und ruft eine Verletzung von Kollagenfasern mit nachfolgender Stimulation einer Neukollagenbildung hervor (Palomar 1540 Fractional Laser, Candela GentleMAX, Candela Smoothbeam) [2]. Dieses Verfahren bietet die Möglichkeit, praktisch beliebige Hautbereiche zu bearbeiten. Die Mängel des Verfahrens sind ein niedriger Heileffekt und eine nicht zufriedenstellende Optik. Die synthetisierte Kollagenmenge reicht nicht aus, um einen gewünschten Verjüngungseffekt zu erreichen. Unter Verjüngungseffekt wird dabei eine beobachtete Verkleinerung der Faltengröße verstanden. Nach diesem Verfahren wird die Hautfarbe dank der verbesserten Kapillardurchblutung verbessert und ein temporäres kutanes Ödem hervorgerufen, was einen provisorischen Effekt der Faltenverkleinerung schafft.

Aus dem Stand der Technik sind Hautverjüngungsverfahren unter Einsatz von nichtinvasiver Ultraschallbestrahlung bekannt [3, 4, 5]. Die genannten Verfahren ermöglichen eine Behandlung von verschiedenen Hautbereichen in vorgegebenen Einwirkungszonen. Diese Verfahren sind dadurch bemängelt, dass die Gewebe durch eine Ultraschallwellenfront beansprucht werden. Folglich wird nicht nur die vorgegebene Zone behandelt, sondern auch die Umgebungsgewebe werden betroffen. Das führt zu einer Vergrößerung der Gewebeverletzungszone und verzögert die Rehabilitation.

Der nächstkommende Stand der Technik gegenüber dem erfindungsgemäßen Verfahren ist ein Verfahren einer mikroablativen Fotoverjüngung der Haut [6]. Das Wesen dieses Verfahrens besteht darin, dass die Haut mit Laser bestrahlt wird. Bei dieser Einwirkung handelt es sich nicht um einen breiten Laserstrahl sondern um zahlreiche Mikrobündel. Dabei bewirkt jeder der Mikrobündel entweder eine Koagulation und Verdampfung oder eine Ablation der Hautmikrobereiche je nach den spektralen und zeitlichen Kenngrößen der benutzten Strahlung. Die Mikrobündel können 1 bis einige Hunderte Mikrometer im Durchmesser haben und zueinander bis einige Hunderte Mikrometer abstehen. Dem Heileffekt nach diesem Verfahren liegt die Annahme zugrunde, dass die entfernten oder zerstörten Gewebe durch neue Zellen ersetzt werden. Nach einigen Behandlungen sollte es möglich sein, die gesamte Haut durch die neue Haut im Behandlungsbereich zu ersetzen. Wird ein Mikrokoagulationseffekt der Haut unter Einsatz von Gläsern mit ER-Laser benutzt (Lasergerät "fraxel", Wellenlänge 1,54 µm), so kommt die Hitzezersetzung der Hautzellen ohne Verdampfung zustande. Wird eine solche Strahlung benutzt, die sowohl eine Verdampfung als auch eine Koagulation der Hautzellen bewirken kann (z. B. CO2-Gaslaserstrahlung, Wellenlänge 10,64 µm), so entstehen Mikrokanäle des verdampften Gewebes mit einer sie umgebenden Koagulationszone. Wird eine ER-Laserstrahlung (mit 2,94 µm langen Wellen) benutzt, so kommt die Gewebeverdampfung in Form von Mikrokanälen ohne Koagulation der umgebenden Gewebe zustande. Dieses bekannte Verfahren hat folgende Mängel:
- Die Tiefe der Mikroverletzungen, in der ein Verjüngungseffekt erreicht werden kann, ist durch die Tiefe der Koagulation oder Ablation begrenzt. Folglich ist es nicht möglich, die Verjüngung tief in der Haut (Derma) und der Unterhaut (Hypodermis) auszuführen.
- Aufgrund der geringen Tiefe der Mikroverletzungen ist es nicht möglich, eine intensive Geweberegeneration während der Behandlung der trophischen und eitrigen Wunden usw. zu bewirken; das heißt, dass das Verfahren nicht ge-eignet ist, wenn eine Gewebeerneuerung ganz tief durchgeführt werden muss.
- Bei diesem Verfahren handelt es sich um ein invasives Verfahren, was ein Ansteckungsrisiko der einer Einwirkung ausgesetzten Oberfläche erhöht.
- Infolge der Gewebeentfernung kommt es zu einem Kontakt zwischen den lebendigen Geweben und der Umgebung. Das kann ein Wachstum von fibrösen Geweben anstelle einer vollständigen Erneuerung der isomorphen Gewebe verursachen.
- Die anhand der mikroablativen Methoden durchgeführten Behandlungen sind schmerzhaft und setzen somit eine Anwendung eines Anästhetikums voraus.

Die US 5,725,482 beschreibt ein Verfahren zum Fokussieren von Schwingungsenergie auf ein Zielvolumen innerhalb eines umgebenden, angrenzenden Mediums überträgt Energie hoher Intensität von Energiequellen mit niedrigem Niveau auf das Zielvolumen. Mehrere stehende Kompressionswellen werden im Medium entlang entsprechender Längsachsen zwischen gegenüberliegenden Paaren von koordinierten Wandlern erzeugt. Das Zielvolumen befindet sich am gemeinsamen Schnittpunkt der Achsen der stehenden Kompressionswellen. Die gegenüberliegenden Paare von Messwandlern sind in einem Abstand voneinander angeordnet, der einem ganzzahligen Vielfachen der halben Wellenlänge der entsprechenden stehenden Welle entspricht. Der Phasenwinkel jeder stehenden Kompressionswelle wird so eingestellt, dass jede Welle ihre maximale Intensität (Amplitude) innerhalb des Zielvolumens an dem Punkt erreicht, an dem sie sich mit den anderen stehenden Wellen überschneidet. Die mehreren sich überschneidenden stehenden Wellen interferieren konstruktiv im Zielvolumen, wodurch dem Zielvolumen eine intensivere Schwingungsenergie verliehen wird als dem umgebenden Medium.

US 2005/0085748 A1 beschreibt eine Vorrichtung und ein Verfahren zur Verwendung von mit Mikrobläschen, thrombolytischen Medikamenten oder anderen Wirkstoffen angereichertem Ultraschall zur Gerinnselauflösung, wobei mindestens ein Ultraschallwandler eine Vielzahl von akustischen Signalen erzeugt und Zeit-, Amplituden-, Phasen- und Frequenzmodulation der Signale eine gleichmäßigere Leistungsabgabe mit weniger Lücken im Ultraschallfeld ermöglichen. Die Interferenzmuster von einem oder mehreren Schallköpfen werden ständig in ihrer Position verschoben. Ein phasengesteuertes Array von Schallköpfen kann einen Strahl erzeugen, der über den zu behandelnden Bereich geschwenkt wird. In einer anderen Ausführungsform kann ein Array von Schallköpfen Ultraschall mit einer Reihe von leicht variierenden Frequenzen erzeugen, um ein Interferenzmuster zu erzeugen, das durch das zu behandelnde Gewebe ein- und ausschwingt. durch das Zielgewebe streicht. Ein einziges Array kann verwendet werden, um beide Effekte gleichzeitig oder getrennt zu erzeugen.

Es ist Aufgabe der Erfindung, eine Vorrichtung für eine nichtinvasive biologische Gewebeerneuerung zu entwickeln, wobei die Leistungsmerkmale, die Eigenschaften und die Strukturen der Gewebe wiederhergestellt werden. Dafür werden Mikroverletzungsbereiche in vorgegebenen Zonen der biologischen Gewebe erzeugt, wobei eine nachfolgende natürliche Regeneration der jeweiligen biologischen Gewebe in den genannten Zonen ermöglicht wird. Dabei müssen Mikroverletzungen (Mikrozerstörungen) im biologischen Gewebe erzeugt werden, ohne dass Mikrokanäle gebildet werden, die mit einer aggressiven Umgebung kontaktieren. Das wird dadurch ermöglicht, dass ein Wachstum fibrösen Gewebes völlig vermieden wird. Eine solche Einwirkung soll es ermöglichen, die Zonen der Mikroverletzungen mit einer nachfolgenden Regeneration sowohl von Oberflächen- als auch von tiefliegendem biologischem Gewebe beliebiger Art und mit beliebiger Lokalisation zu bilden. Darüber hinaus soll Eine Anwendung der Vorrichtung eine Minderung von Schmerzempfindungen und eines Ansteckungs-risikos im Vergleich zu anderen aus dem Stand der Technik bekannten, darunter auch mikroablativen, Verfahren sicherstellen.

Die gestellte Aufgabe wird durch die Merkmale des Anspruchs 1 gelöst.

Die gestellte Aufgabe wird mittels der erfindungsgemäßen Vorrichtung zur biologischen Gewebeerneuerung, Wiederherstellung der Leistungsmerkmale, der Eigenschaften und der Strukturen dieser Gewebe gelöst. Bei Anwendung werden Mikroverletzungsbereiche mit der Möglichkeit einer nachfolgenden natürlichen Regeneration der jeweiligen biologischen Gewebe in vorgegebenen Bereichen der biologischen Gewebe gebildet. Dafür werden die Gewebe einer mechanischen Verletzung mit vorgegebenem Schwere-grad ausgesetzt, indem wenigstens ein Interferenzgebiet akustischer Wellen in vor-gegebenen Bereichen erzeugt wird. Die akustischen Wellen gehen von zwei Quellen aus und breiten sich in den zu erneuernden Geweben aus.

Im allgemeinen Fall werden mittels der erfindungsgemäßen Vorrichtung starke akustische phasengleiche Wellen auf der Oberfläche eines verjüngungsbedürftigen oder eines höher- liegenden Gewebes erzeugt. Diese akustischen phasengleichen Wellen haben Solleigenschaften (Auslegungseigenschaften), insbesondere eine Sollleistung. Insbesondere eine Leistungsveränderung der akustischen Wellen ermöglicht es, dementsprechend die vorgegebene Tiefe der Mikroverletzungsbereiche zu verändern. Durch die Nutzung von Interferenz der zusammenwirkenden akustischen Wellen können die Abmessungen vermindert und die Lokalisation in allen Richtungen des Mikroverletzungsgebiets der Gewebe ermittelt werden. Das erlaubt es, die Effizienz der Richtwirkung wesentlich zu steigern und dabei die Rehabilitationsdauer zu verkürzen. Die Mikroverletzung der Gewebe nach dem erfindungsgemäßen Ver-fahren ist von nicht thermischem Charakter. Aus diesem Grund kommt bei der Ein-wirkung auf die biologischen Gewebe weder ihre Verdampfung noch Koagulation zustande. Außerdem sind die akustischen Wellen fähig, in die biologischen Gewebe auf eine bestimmte Tiefe einzudringen, ohne dass jegliche Kanäle gebildet werden (diese Tiefe wird durch die Eigenschaften der akustischen Wellen festgelegt). Somit wird der Kontakt zwischen den lebendigen Geweben und Sauerstoff infolge dieser Einwirkung nicht verlängert. Das verringert das Risiko eines fibrösen Gewebewachstums und lässt die Annahme zu, dass es sich in diesem Fall um eine tatsächliche und keine visuell beobachtete biologische Erneuerung/Verjüngung der biologischen Gewebe handelt. Die Energie der akustischen Wellen nimmt in den Interferenzzonen mit vorgegebener Lokalisation zu. Unter der Einwirkung der akustischen Wellenenergie werden in diesen Zonen Bereiche eines verletzten und nicht zerstörten Gewebes gebildet. Die Regeneration der biologischen Gewebe kann nicht nur infolge einer vollständigen Zerstörung der Gewebezellen sondern auch durch ihre teilweise Verletzung zustande kommen. Dadurch erfolgt die biologische Gewebeerneuerung in den Gebieten mit einer vorgegebenen Lokalisation. Da es zu keiner vollständigen Zerstörung der tief liegenden Zellen der biologischen Gewebe kommt, kann auch die Rehabilitationsdauer wesentlich verkürzt werden. Die Dolenz bei der Einwirkung ist auch wesentlich vermindert worden, weil der Verletzungsbereich kleiner ist, der Verletzungsgrad vorgegeben werden kann und weil es keine Wärmeeinwirkung gibt.

Die Fläche der Quellen der phasengleichen akustischen Wellen liegt vorzugsweise im Bereich zwischen 10 nm2 und 10 µm2. Alle Nullpunkte (Zentren/Epizentren) der akustischen Wellen werden vorteilhaft im gleichen Abstand zueinander angeordnet. Diese Abstände werden in einem Bereich zwischen 10 µm und 1 cm gewählt.

Mittels der erfindungsgemäßen Vorrichtung werden die mechanischen Verletzungsbereiche der Gewebe vorteilhaft unterhalb der Oberfläche des Gewebes ausgebildet, welches im Kontakt mit der Umgebung steht, ohne dass die Kontaktfläche zwischen den lebendigen Geweben und den aggressiven Medien vergrößert wird. Somit ist nach der Erfindung eine Vorrichtung bereitgestellt, mittels welcher Mikroverletzungsbereiche erzeugt werden können, ohne dass die Berührungsfläche zwischen den lebendigen Geweben und der Umgebung vergrößert werden muss. Dadurch kann das Ansteckungsrisiko während der Rehabilitation im Vergleich zu den bekannten Verfahren wesentlich vermindert werden.

Die Mindestleistung der erzeugten akustischen Wellen wird so gewählt, dass:
- die Leistung einer von einem Zentrum/Epizentrum (Nullpunkt) ausgehenden Einzelwelle nicht ausreichend ist, um die einer Einwirkung auszusetzenden biologischen Gewebe mechanisch zu verletzen/zu zerstören;
- die bei einer Interferenz der von Nebenzentren ausgehenden Wellen erreichte Gesamtleistung dafür ausreicht, um die einer Einwirkung auszusetzenden biologischen Gewebe mit einem vorgegebenen Schweregrad mechanisch zu verletzen.

Das nach der Einwirkung auf die Hautoberfläche entstehende Erythem kann zur visuellen Kontrolle einer ausreichenden Einwirkung benutzt werden.

Die Frequenz ist eine weitere Eigenschaft der akustischen Wellen, deren Veränderung das Ergebnis der Einwirkung auf das biologische Gewebe variieren kann. So entsteht bei der Auswahl der Frequenz der akustischen Wellen je nach Eigenschwingungsfrequenz der jeweiligen biologischen Gewebe die Möglichkeit, auf konkrete biologische Gewebe selektiv regenerierend einzuwirken, indem eine Resonanz mit den erwähnten Frequenzen erreicht wird. Somit wird eine solche Frequenz der akustischen Welle aus der Reihe der vorteilhaften Ausgestaltungen der Einrichtungen gemäß der Eigenfrequenz des verjüngungsbedürftigen biologischen Gewebes gewählt, welche eine selektive Einwirkung auf das genannte verjüngungsbedürftige biologische Gewebe sicherstellt.

Bei vorteilhaften Ausgestaltungen der Einrichtung wird der Grad der mechanischen Verletzung aus einem Bereich gewählt, welcher zwischen zwei Niveaus liegt, und zwar dem einer Zerstörung der Zellmembranintegrität und dem einer Volldestruktion der Zellen des verjüngungsbedürftigen biologischen Gewebes. Dadurch erlaubt die Einwirkung, eine Regeneration der biologischen Gewebe sowohl mit einer Destruktion der ganzen (intakten) Zellen dieser Gewebe als auch ohne Destruktion zu stimulieren. Dabei kann ein Erneuerungs-/Verjüngungseffekt sogar bei einer teilweisen Verletzung der Zellen der biologischen Gewebe erreicht werden.

Bei anderen vorteilhaften Ausgestaltungen werden die akustischen Wellen in Form von akustischen Richtwellen erzeugt. Dadurch wird es auch möglich, die Bildung der Mikroverletzungsbereiche zu lokalisieren und zu optimieren.

Die gestellte Aufgabe wird auch anhand der Ausgestaltungen einer Einrichtung zur Ausführung des oben beschriebenen erfindungsgemäßen Verfahrens zu einer biologischen Gewebeerneuerung gelöst. Mittels dieser Vorrichtung kann die Wiederherstellung der Leistungsmerkmale, der Eigenschaften und der Strukturen von Geweben erfolgen. Die Einrichtung enthält eine Strahlungsquelle sowie ein Mittel zur Bildung von zahlreichen Zentren/Epizentren von akustischen Wellen auf der Oberfläche der erneue-rungsbedürftigen oder der höherliegenden biologischen Gewebe.

In einer ersten Ausgestaltung der Einrichtung wird die gestellte Aufgabe dadurch gelöst, dass die Strahlungsquelle in Form einer Ultraschallquelle ausgebildet ist. Die Ultraschallquelle hat ein Mittel zur Bildung von zahlreichen Zentren/Epizentren von gleich zueinander beabstandeten akustischen Wellen auf der Oberfläche der erneuerungsbedürftigen oder der höherliegenden biologischen Gewebe.

Bei der ersten und der zweiten Ausgestaltung der Einrichtung ist es vorteilhaft, dass der Abstand zwischen den Zentren/Epizentren der akustischen Wellen im Bereich zwischen 10 µm und 1 cm liegt.

Die oben genannten sowie alle anderen Vorteile der erfindungsgemäßen Vorrichtung zur biologischen Gewebeerneuerung und Wiederherstellung der Leistungsmerkmale, Eigenschaften und Strukturen der Gewebe, sowie die Vorteile der Ausgestaltungen der entsprechenden Einrichtung werden eingehend an einem Beispiel von möglichen vorteilhaften Ausgestaltungen und anhand der Figuren mit jeweiligen Bezugszeichen weiter unten betrachtet.

Diese Ausgestaltungen sind allerdings nicht auf die hier beschriebenen Fälle beschränkt. Es zeigen:
- Fig. 1: ein Übersichtsbild zur Bildung von Mikroverletzungsbereichen der Zellen im biologischen Gewebe gemäß einer der möglichen Ausgestaltungen,
- Fig. 2: ein Übersichtsbild zur Bildung von Mikroverletzungsbereichen der Zellen im biologischen Gewebe gemäß einer zweiten Ausgestaltung,
- Fig. 3: ein Übersichtsbild zur Bildung von Mikroverletzungsbereichen der Zellen im biologischen Gewebe gemäß einer dritten Ausgestaltung und
- Fig. 4: eine schematische Darstellung der Einrichtung nach der dritten Ausgestaltung.

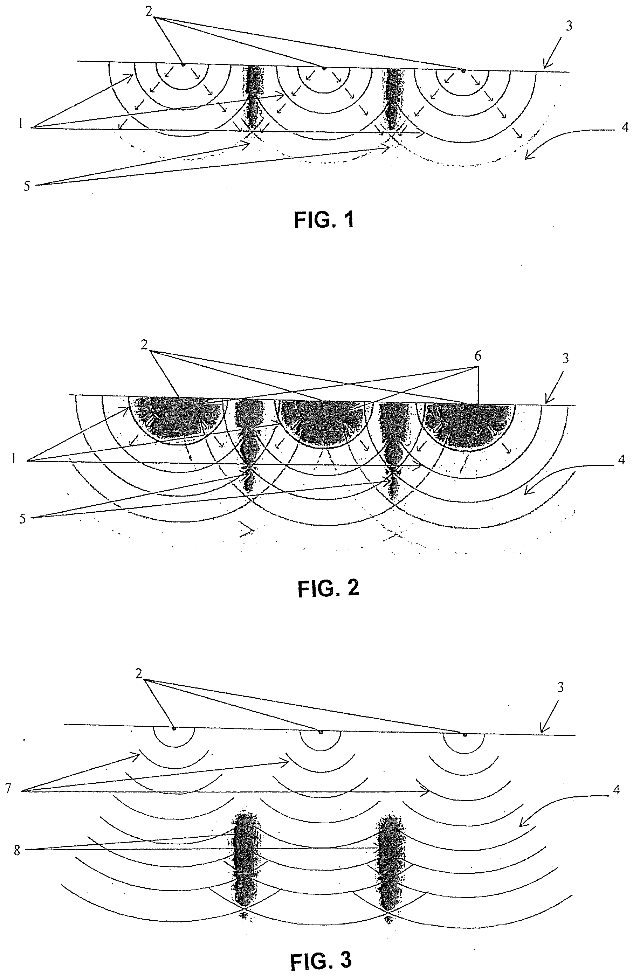

Fig. 1 weist ein Übersichtsbild zur Bildung von Mikroverletzungsbereichen der Zellen im biologischen Gewebe gemäß einer der möglichen Ausgestaltungen auf. Hier breiten sich akustische Wellen 1 von entsprechenden Zentren/Epizentren (Nullpunkten) 2 auf einer Oberfläche 3 eines biologischen Gewebes 4 ins Innere des biologischen Gewebes 4 aus. Dabei ist die Leistung jeder der akustischen Wellen 1 so gewählt, dass diese Leistung nicht ausreicht, um jegliche Bestandteile des zu behandelnden biologischen Gewebes 4 mechanisch zu zerstören. Die Mikroverletzungsbereiche werden nur in den Interferenzzonen 5 (in der Figur dunkel gekennzeichnet) der akustischen Wellen 1 entstehen, welche den benachbarten Zentren/Epizentren 2 entspringen.

Fig. 2 zeigt ein Übersichtsbild zur Bildung von Mikroverletzungsbereichen der Zellen im biologischen Gewebe gemäß einer zweiten der möglichen Ausgestaltungen. Hier breiten sich die akustischen Wellen 1 von den entsprechenden Zentren/Epizentren 2 auf der Oberfläche 3 des biologischen Gewebes 4 ins Innere des biologischen Gewebes 4 aus. Dabei ist die Leistung jeder der akustischen Wellen 1 so gewählt, dass sie mechanische Zerstörungsbereiche 6 des zu behandelnden biologischen Gewebes 4 neben dem jeweiligen Zentrum/Epizentrum 2 (Volldestruktion der Oberflächengewebe) und Mikroverletzungsbereiche (Bereiche mit lokalen Verletzungen) in den Interferenzzonen 5 der den benachbarten Zentren/Epizentren 2 entspringenden akustischen Wellen 1 bilden wird. Die mechanischen Zerstörungsbereiche 6 und die Interferenzzonen 5 sind in der Figur dunkel gekennzeichnet.

Fig. 3 zeigt ein Übersichtsbild zur Bildung von Mikroverletzungsbereichen der Zellen im biologischen Gewebe nach einer dritten der möglichen Ausgestaltungen. Hier breiten sich akustische Wellen 1 von den jeweiligen Zentren/Epizentren 2 auf der Oberfläche 3 des biologischen Gewebes 4 ins Innere des biologischen Gewebes 4 aus. Um die Mikroverletzungsbereiche in den tief liegenden Geweben zu bilden, werden die akustischen Wellen in Form von akustischen Richtwellen 7 erzeugt. Die Mikroverletzungsgebiete bilden sich in den Interferenzzonen 8 aller zwei akustischen Richtwellen 7 von den entsprechenden benachbarten Zentren/Epizentren 2. Die Interferenzzonen 8 sind in der Figur dunkel gekennzeichnet.

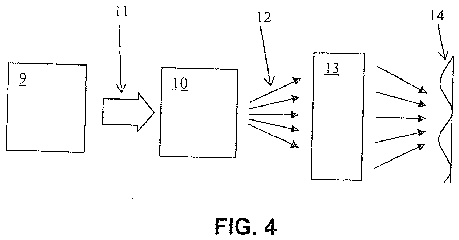

Fig. 4 zeigt eine schematische Darstellung der Einrichtung einer nicht zur Erfindung gehörigen Ausführungsform. Hier ist die Strahlungsquelle in Form einer Laserstrahlungsquelle 9 ausgebildet. Das Mittel zur Bildung von zahlreichen Zentren/Epizentren der akustischen Wellen auf der Oberfläche der erneuerungsbedürftigen oder der höherliegenden biologischen Gewebe enthält einen Lichtteiler 10 und ein optisches System 13. Der Lichtteiler 10 teilt das Ausgangslaserbündel 11 in so viele Laserbündel 12 auf, die erforderlich sind, um die vorgegebene Verteilung zu erreichen. Das optische System 13 bringt die mit dem Lichtteiler 10 erzeugten Laserbündel 12 unter solchen Winkeln zusammen, die erforderlich sind, um eine vorgegebene räumliche Strahlintensitäts-verteilung 14 mittels einer Mehrstrahlinterferenz zu bekommen.

Die angemeldete Vorrichtung funktioniert wie folgt:
Die akustischen Wellen 1 (7) mit vorgegebenen Eigenschaften (Leistung, Frequenz) werden mit Hilfe einer der Ausgestaltungen der erfindungsgemäßen Einrichtung zur biologischen Gewebeerneuerung mit Wiederherstellung der Leistungsmerkmale, der Eigenschaften und der Strukturen der Gewebe erzeugt. Jede akustische Welle 1 (7) fängt an, sich vom jeweiligen Zentrum/Epizentrum 2 aus ins Innere des biologischen Gewebes 4 auszubreiten.

So reicht z. B. die Mindestleistung der akustischen Welle 1 bei der Ausgestaltung nach Fig. 1 nicht aus, um jegliche Bestandteile des behandelbaren biologischen Gewebes 4 mechanisch zu zerstören. Deswegen ist die Ausbreitung der Welle 1 von keiner Destruktion des biologischen Gewebes 4 begleitet. Jedoch ermöglicht die Interferenz mit den aus den benachbarten Zentren/Epizentren 2 entspringenden akustischen Wellen 1, die Gesamtleistung der akustischen Welle 1 lokal so weit zu steigern, bis diese Leistungswerte ausreichen, um die Gebiete mit mechanischen Verletzungen eines bestimmten Verletzungsgrads der biologischen Gewebe 4 in diesen Interferenzzonen 5 zu bilden. Dabei werden sich diese Gebiete von der Oberfläche 3 ins Innere des Gewebes ausbreiten und Zonen mit ungleichmäßiger Mikroverletzung (eines vorgegebenen Verletzungsgrads) der Zellen im biologischen Gewebe 4 darstellen.

Eine solche Verletzung der Gewebe setzt keine Vergrößerung der Kontaktfläche zwischen den lebendigen Geweben und der aggressiven Umgebung voraus, was das Wachstum des fibrösen Gewebes minimiert.

Nimmt die Leistung der akustischen Wellen 1 zu, so können solche Leistungswerte nach Ausgestaltung gemäß Fig. 2 erreicht werden, bei denen jede akustische Einzelwelle 1 fähig ist, eine unabhängige Destruktion der Gewebe hervorzurufen. Dabei wird sich der Einwirkungsprozess etwas ändern und wie folgt aussehen. Die Wellen 1 gehen von jedem Zentrum/Epizentrum 2 aus und dringen ins Innere des biologischen Gewebes 4 ein. Sie verursachen eine mechanische Zerstörung (Zonen 6 der mechanischen Zerstörung), bis die Leistungswerte dieser Wellen unter ihre Schwellenwerte sinken. Die weitere Ausbreitung der Wellen 1 ins Innere des biologischen Gewebes 4 wird von keiner Gewebezersetzung begleitet bis auf die Interferenzzonen 5 der Wellen 1, welche von den benachbarten Zentren/Epizentren 2 ausgehen. Somit handelt es sich beim Verletzungsgebiet um einen Volldestruktionsbereich des Oberflächengewebes (Zonen 6 der mechanischen Zerstörung) und um Bereiche mit lokalen Verletzungen (Interferenzzonen 5).

Der visuell beobachtete Effekt wird sich in diesem Fall dadurch bemerkbar machen, dass der sogenannte "Frost" auf der zu behandelnden Oberfläche entsteht. Beim "Frost"-Effekt handelt es sich um Gebiete mechanisch zerstörten Gewebes. Dabei ermöglicht die Leistungsvariation der akustischen Welle, auch die Tiefenlage der Mikroverletzungsbereiche zu variieren.

Mittels der erfindungsgemäßen Vorrichtung können akustische Richtwellen 7 erzeugt werden. In diesem Fall wird die Interferenz der akustischen Wellen 7 nur in der Tiefe der biologischen Gewebe zustande kommen, ohne dass die Oberflächenschichten verletzt werden. Die Gebiete der me-chanischen Mikroverletzung werden in den Interferenzzonen 8 entstehen.

Die Regeneration der Gewebe nach Einsatz der erfindungsgemäßen Vorrichtung wird im Vergleich zum Prototyp schneller verlaufen, denn sogar bei der Einwirkung nach der zweiten Ausgestaltung (s. Fig. 2) werden nur Oberflächengewebe (Zonen 6 der me-chanischen Zerstörung) eine vollständige mechanische Destruktion erfahren. Die tief im Gewebe liegenden Zellen werden nicht vollständig zerstört, sondern nur verletzt (Interferenzzonen 5). Bei allen anderen Ausgestaltungen einschließlich beliebiger hier nicht näher betrachteter Ausgestaltungen liegt überhaupt keine Volldestruktion jeglicher biologischer Gewebe vor.

Die erfindungsgemäße Einrichtung zur biologi-schen Gewebeerneuerung kann auch verschiedene Ausgestaltungen haben.

Die Einrichtung nach der ersten Ausgestaltung kann als Strahlungsquelle eine leistungsstarke Ultraschallquelle aufweisen. Als ein "Erzeuger" der Zentren/Epizentren 2 der akustischen Wellen 1 (7) kann eine Kontaktfläche benutzt werden. Die Kontaktfläche wird in Form eines geordneten Nadelsatzes ausgebildet. Die Nadeln werden in einem vorgegebenen Abstand zueinander angeordnet (der Nadelabstand liegt im Bereich zwischen 10 µm und 1 cm) und berühren die Oberfläche 3 des biologischen Gewebes 4 über 10 nm2 bis 10 µm2 große Flächen.

Der Übersichtsplan ist Fig. 4 zu entnehmen. Das durch die Laser-quelle 9 erzeugte breite Laserbündel 11 fällt auf einen Lichtteiler 10 ein. Der Lichtteiler 10 teilt das Laserbündel 11 in mehrere Laserbündel 12 auf. Dabei handelt es sich um eine solche Menge von Laserbündeln 12, welche erforderlich ist, um eine vorge-gebene Verteilung zu erreichen. Das optische System 13 bringt die anhand des Lichtteilers 10 erzeugten Laserbündel 12 unter solchen Winkeln zusammen, die erforderlich sind, um eine vorgegebene räumliche Bündelintensitätsverteilung 14 mittels der Mehrstrahlinterferenz zu erreichen.

Die erfindungsgemäße Einrichtungen zur Erneuerung/Verjüngung der biologischen Gewebe sind in der medizinischen Praxis bekannt. Dennoch zeigt die oben angeführte Beschreibung samt den Beispielen, welche einige nicht erschöpfende Ausgestaltungen der Erfindung schildern, dass es das erfindungsgemäße Verfahren und die Einrichtung hierfür ermöglichen, neue unerwartete technische Ergebnisse zu erreichen. Das hängt vor allem damit zusammen, dass der Erneuerungseffekt verschiedener biologischer Gewebe, die unterschiedlich tief liegen, dadurch geschaffen werden kann, dass die Mikroverletzungsgebiete ohne thermische Einwirkung, d. h. ohne Verdampfung oder Koagulation aller höherliegenden Gewebe gebildet werden können. Somit kommt die Regeneration der Gewebe ohne jegliches Wachstum fibröser Zellen zustande. Folglich liegt eine tatsächliche und nicht nur visuell beobachtete Verjüngung vor.

### Informationsquellen

1. B. Yeremeyev, K. Kalaydzyan. Laser gegen Falten. Elektronischer Almanach. "Kosmetik und Medizin". [Elektronische Informationsressource] - 6. April 2012 - http://daniel.ru/cm/arc/r403.htm.
2. Palomar-Verjüngung. Web-Site des GesundheitsZentrum/Epizentrums RO-DEN [Elektronische Informationsressource] - 4. Mai 2012 - http://www.roden.by/cosmetology/palomar-omolojenie/.
3. Patentanmeldung US 2011/0218464 A1, veröffentlicht am 08.09.2011.
4. Patentanmeldung US 2012/0016239 A1, veröffentlicht am 19.01.2012.
5. Patentanmeldung US 2012/0053458 A1, veröffentlicht am 01.03.2012.
6. Patent US 6,997,923 B2, veröffentlicht am 31.10.2002.

## Patentansprüche

1. Einrichtung zur biologischen Gewebeerneuerung der Haut mit Wiederherstellung der Leistungsmerkmale, der Eigenschaften und der Strukturen von Geweben, wobei sie eine Ultraschallquelle sowie ein Mittel zur Bildung von zahlreichen Epizentren (2) akustischer, nicht-thermischer, phasengleicher Wellen (1) auf der Oberfläche (3) der erneuerungsbedürftigen Haut aufweist, wobei diese Epizentren (2) im gleichen Abstand zueinander liegen, und das Mittel ausgebildet ist, die Wellen von den Epizentren halbkreisförmig auszusenden, um ein Interferenzgebiet der Wellen bereitzustellen, **dadurch gekennzeichnet, dass** der Abstand zwischen den Epizentren (2) der akustischen Wellen (1) im Bereich zwischen 10 µm und 1 cm liegt und die Fläche der Quellen der phasengleichen akustischen Wellen an dem Mittel im Bereich zwischen 10 nm² und 10 µm² liegt.

## Claims

1. A device for the biological renewal of skin tissue with restoration of the performance characteristics, properties and structures of tissues, having an ultrasound source and a means for forming numerous epicenters (2) of acoustic, non-thermal, in-phase waves (1) on the surface (3) of the skin in need of renewal, these epicentres (2) being equidistant from one another, and the means being designed to emit the waves from the epicentres in a semicircle in order to provide an interference area for the waves , **characterized in that** the distance between the epicentres (2) of the acoustic waves (1) is in the range between 10 µm and 1 cm and the area of the sources of the in-phase acoustic waves at the means is in the range between 10 nm2 and 10 µm2.

## Revendications

1. Dispositif pour le renouvellement biologique des tissus de la peau avec la récupération des caractéristiques de performance, des propriétés et des structures des tissus où il présente une source d'ultrasons ainsi qu'une ressource de formations de nombreux épicentres (2) d'ondes acoustiques, non thermiques et équiphasées (1) sur la surface (3) de la peau ayant besoin de renouvellement où ces épicentres (2) se situent à titre parallèle à égale distance et la ressource est formée de sorte que les ondes sont à émettre des épicentres et ce à titre semi-circulaire pour fournir un dispositif d'interférence des ondes est caractérisé de sorte que la distance entre les épicentres (2) des ondes acoustiques (1) se trouve dans la zone entre 10 µm et 1 cm et la surface des sources des ondes équiphasées acoustiques se situe sur le milieu dans la zone entre 10 nm² et 10 µm².
